# EUROPEAN PATENT SPECIFICATION

(11) **EP 1 251 871 B1**
(45) Date of publication and mention of the grant of the patent: **23.04.2008**
(21) Application number: 01903625.0
(22) Date of filing: 05.01.2001
(51) Int. Cl.: A61K 39/39, A61K 39/002, A61K 39/02, A61K 39/12, A61P 35/00, A61P 37/00, A61K 31/722, A61K 31/7125, C07K 14/34

(54) **PHARMACEUTICAL COMPOSITION FOR IMMUNOMODULATION AND PREPARATION OF VACCINES COMPRISING AN ANTIGEN AND AS ADJUVANTS AN IMMUNOGENIC OLIGODEOXYNUCLEOTIDE AND A POLYCATIONIC POLYPEPTIDE**
PHARMAZEUTISCHE ZUSAMMENSETZUNG ZUR IMMUNSTIMULATION UND ZUR ZUBEREITUNG VON IMPFSTOFFEN ENTHALTEND EIN ANTIGEN, UND ALS ADJUVANTIEN EIN IMMUNOGENES OLIGODESOXYNUKLEOTID UND EIN POLYKATIONISCHES POLYPEPTID
COMPOSITION PHARMACEUTIQUE POUR IMMUNOMODULATION ET PREPARATION DE VACCINS COMPRENANT UN ANTIGENE, ET COMME ADJUVANTS UN OLIGODESOXYRIBONUCLEOTIDE IMMUNOGENE ET UN POLYPEPTIDE POLYCATIONIQUE

(30) Priority: 28.01.2000 AT 1292000
(43) Date of publication of application: 30.10.2002
(73) Proprietor: Intercell AG, 1030 Wien (AT)
(72) Inventor: LINGNAU, Karen, A-1130 Vienna (AT); MATTNER, Frank, A-1190 Vienna (AT); SCHMIDT, Walter, A-1030 Vienna (AT); BIRNSTIEL, Max, CH-6953 Lugaggia (CH); BUSCHLE, Michael, A-2380 Perchtoldsdorf (AT)
(74) Representative: Alge, Daniel
(86) International application number: PCT/EP2001/000087
(87) International publication number: WO 2001/054720

(56) References cited:
- WO-A-97/30721
- WO-A-99/38528
- US-A- 5 109 026
- BUSCHLE M ET AL: "Transloading of tumor antigen-derived peptides into antigen-presenting cells" PROCEEDINGS OF THE NATIONAL ACADEMY OF SCIENCES OF USA,US,NATIONAL ACADEMY OF SCIENCE. WASHINGTON, vol. 94, 1 April 1997 (1997-04-01), pages 3256-3261, XP002086009 ISSN: 0027-8424
- BUSCHLE M ET AL: "DEVELOPMENT OF DEFINED, SYNTHETIC VACCINES BY IN VIVO CHARGING OF ANTIGEN PRESENTING CELLS WITH ANTIGEN" JOURNAL OF INVESTIGATIVE DERMATOLOGY,NEW YORK, NY,US, vol. 114, no. 1, January 2000 (2000-01), page 235 XP000996450 ISSN: 0022-202X
- GALL D ET AL: "ADJUVANT ACTIVITY OF POLYELECTROLYTES" IMMUNOLOGY,OXFORD,GB, vol. 23, no. 4, 1972, pages 569-575, XP000995977 ISSN: 0019-2805
- SCHMIDT ET AL.: "Cell-free tumor antigen peptide-based cancer vaccines" PNAS, vol. 94, April 1997 (1997-04), pages 3262-3267, XP002168512
- MCCLUSKIE M J ET AL: "NOVEL STRATEGIES USING DNA FOR THE INDUCTION OF MUCOSAL IMMUNITY" CRITICAL REVIEWS IN IMMUNOLOGY,CRC PRESS, INC,XX, vol. 19, no. 4, 1999, pages 303-329, XP000995880 ISSN: 1040-8401
- TOSSI ET AL: 'Amphipathic, alpha-helical antimicrobial peptides.' BIOPOLYMERS vol. 55, no. 30, 2000, pages 4 - 30
- MCCLUSKIE; DAVIES: 'CpG DNA as mucosal adjuvant' VACCINE vol. 18, 2000, pages 231 - 237
- DALLAS; FALKOW: 'Amino acid sequence homology between cholera toxin and Escherichia Coli heat-labile toxin' NATURE vol. 288, no. 4, December 1980, pages 499 - 501

## Description

The invention relates to a pharmaceutical composition, e.g. to be used for immunomodulation especially as vaccines.

Vaccines can save more lives (and resources) than any other medical intervention (Nossal, 1998). Owing to world-wide vaccination programs the incidence of many fatal diseases has been decreased drastically. Although this notion is valid for a whole panel of diseases, e.g. tuberculosis, diphtheria, pertussis, measles and tetanus, there are no effective vaccines for numerous infectious disease including most viral infections, such as AIDS. There are also no effective vaccines for other diseases, infectious or non-infectious claiming millions the lifes of millions of patients per year including malaria or cancer. In addition, the rapid emergence of antibiotic-resistant bacteria and microorganisms calls for alternative treatments with vaccines being a logical choice. Finally, the great need for vaccines is also illustrated by the fact that infectious diseases, rather than cardiovascular disorders or cancer or injuries remain the largest cause of death and disability in the world (Bloom and widdus, 1998).

From an immunological point of view one major problem in the field of vaccines today is that traditional vaccines (and/or the immune-modulating compounds contained within these preparations) are designed to induce high levels of antibodies (Harlow and Lane, 1988). However, antibodies on their own are not effective in preventing a large number of diseases including most illnesses caused by viruses, intracellular bacteria, certain parasites and cancer. Examples for such diseases are, but are not restricted to, the above-mentioned HIV virus or Plasmodium spec. in case of malaria. In numerous experimental systems it has been shown that the cellular arm of the immune system, including T cells, rather than the humoral arm, is important for these indications. Therefore, novel, innovative technologies are needed to overcome the limitations of conventional vaccines. The focus must be on technologies that reliably induce the cellular immune system, including antigen specific T cells, which recognize molecules expressed on pathogen infected cells. Ideally, vaccines are designed that induce both T cells distinguishing diseased and/or infected cells from normal cells and, simultaneously, antibodies secreted by B cells recognizing pathogens in extracellular compartments.

Several established vaccines consist of live attenuated organism
where the risk of reversion to the virulent wild-type strain exists. In particular in immunocompromised hosts this can be a live threatening scenario. Alternatively, vaccines are administered as a combination of pathogen-derived antigens together with compounds that induce or enhance immune responses against these antigens (these compounds are commonly termed adjuvant), since these subunit vaccines on their own are generally not effective.

Whilst there is no doubt that the above vaccines are valuable medical treatments, there is the disadvantage that , due to their complexity, severe side effects can be evoked, e.g. to antigens that are contained in the vaccine that display crossreactivity with molecules expressed by cells of vaccinated individuals. In addition, exisiting requirements from regulatory authorities, e.g. the World Health Organization (WHO), the Food and Drug Administration (FDA), and their European counterparts, for exact specification of vaccine composition and mechanisms of induction of immunity, are difficult to meet.

Some widely used vaccines are classified in table 1.

**Table 1**

| **Category of vaccine** | **Example** |
|---|---|
| **Whole cell** | |
| Attenuated bacteria or viruses | Bacille Calmette-Guerin |
| | (BCG) (tuberculosis) |
| | Measles |
| | Mumps |
| | Rubella |
| | Oral Polio vaccine (Sabin) |
| Killed bacteria or viruses | Pertussis |
| | Inactivated polio vaccine (Salk) |
| **Subunit** | |
| Toxoid | Diphtheria |
| | Tetanus |
| Capsular polysaccharide | H. influenzae type B |
| Yeast recombinant subunit | Hepatitis B surface protein |

From (Liljeqvist and Stahl, 1999) with modifications.

Antigen presenting cells belong to the innate immune system, which has evolved as a first line host defense that limits infection early after exposure to microorganisms (Hoffmann et al., 1999). Cells of the innate immune sytem recognize patterns or relatively non-specific structures expressed on their targets rather than more sophisticated, specific structures which are recognized by the adaptive immune system (Hoffmann et al., 1999) . Examples of cells of the innate immune system are macrophages and dendritic cells but also granulocytes (e.g. neutrophiles), natural killer cells and others. By contrast, cells of the adaptive immune system recognize specific, antigenic structures, including peptides, in the case of T cells and peptides as well as three-dimensional structures in the case of B cells. The adaptive immune system is much more specific and sophisticated than the innate immune system and improves upon repeat exposure to a given pathogen/antigen. Phylogenetically, the innate immune system is much older and can be found already in very primitive organisms. Nevertheless, the innate immune system is critical during the initial phase of antigenic exposure since, in addition to containing pathogens, cells of the innate immune system, i.e. APCs, prime cells of the adaptive immune system and thus trigger specific immune responses leading to clearance of the intruders. In sum, cells of the innate immune sytem and in particular APCs play a critical role during the induction phase of immune responses by a) containing infections by means of a primitive pattern recognition system and b) priming cells of the adaptive immune system leading to specific immune responses and memory resulting in clearance of intruding pathogens or of other targets (Roitt et al., 1998). These mechanisms may also be important to clear or contain tumor cells.

As mentioned above, cells of the innate immune system recognize patterns expressed on their respective targets. Examples are lipopolysaccharides (LPS) in the case of Gram-negative bacteria, mycobacterial glycolipids, lipoteichoic acids of Gram-positive bacteria, mannans of yeast and double stranded RNAs of viruses (Hoffmann et al., 1999). In addition they may recognize patterns such as altered glycosylations of proteins on tumor cells.

Recent findings describe DNAs of protozoae or lower eukaryotes as a further pattern recognized by the innate (but possibly also by the adaptive) immune system of mammals (and probably most if not all vertebrates) (Krieg, 1996; Lipford et al., 1998).

The immune system recognizes DNA of lower organisms including bacteria probably due to structural and sequence usage differencies between pathogen and host DNA. In particular short stretches of DNA, derived from non-vertebrates or in form of short oligodeoxynucleotides (ODNs) containing nonmethylated cytosine-guanine dinucleotides (CpG) in a certain base context, are targeted (Krieg et al., 1995). CpG motifs are found at the expected frequency in bacterial DNA but are much less frequent in vertebrate DNA (Lipford et al., 1998; Pisetsky, 1999). In addition, non-vertebrate (i.e. bacterial) CpG motifs are not methylated whereas vertebrate CpG sequences are. These differences between bacterial DNA and vertebrate DNA allow vertebrates to recognize non-vertebrate DNA.

Natural CpG-containing DNA, ODNs, as well as thiophosphate-substituted (exchange of thiophosphate residues for phosphate) ODNs containing CpG motifs (CpG-ODN) are not only potent activators of immune cell proliferation and humoral immune responses (Krieg et al., 1995), but also stimulate strong cellular immune responses (reviewed in (Lipford et al., 1998)). DNA/ODNs containing non-methylated CpG motifs can directly activate monocytic cells (dendritic cells, macrophages) and B cells. Likely, natural killer (NK) cells are not directly activated but respond to monocyte-derived IL-12 (interleukin 12) with a marked increase in their IFN-γ production (Chace et al., 1997). In consequence, the induction of monocytes and NK cells by CpG DNA promotes the induction of Th1-type responses and the development of cytotoxic T cells.

Dendritic cells, which represent important antigen presenting cells during primary immune responses, mature in vitro under the influence of CpG-ODN (Hartmann et al., 1999; Sparwasser et al., 1998) and produce large amounts of IL-12, TNF-α, IL-6 and to a lesser extent IL-10 (Klinman et al., 1996; Sparwasser et al., 1998) when exposed to these compounds. Thus, one characteristic effect of bacterial DNA and CpG-ODNs is the induction of Th1 type humoral and cell-mediated responses to protein antigens, which is characterized by it's specific cytokine pattern (Mosmann et al., 1986). ODNs containing CpG motifs have been used as a vaccine adjuvant in mice to enhance for example the antibody response to a tetanus vaccine (Krieg et al., 1998) or to promote a strong antigen-specific Th1 cytokine response in an experimental model of virus infection (Oxenius et al., 1999). CpG DNA is also described as a strong adjuvant for antibody and cytotoxic T lymphocyte (CTL) responses against Hepatitis B virus antigens (Davis et al., 1998). The induction of strong Th1 cytokine and CTL responses indicates that CpG-ODN may also be useful for cancer vaccines using tumor antigens. First published data show that mice immunized with the idiotype from a B lymphoma in combination with a CpG-ODN as an adjuvant displayed prolonged survival (Weiner et al., 1997).

Although CpG ODNs or non-methylated DNA containg CpG motifs are potentially very powerful adjuvants, there is a more sinister side to this technology (Pisetsky, 1997). For example, it has been reported that with high doses of bacterial DNA containing non-methylated CpG motifs septic shock can be induced (Sparwasser et al., 1997) under certain circumstances. This is likely due to excessive amounts of cytokines, in particular TNF-α and IL-6, but also others, that are secreted by cells upon exposure to CpG-ODN or to bacterial DNA (Sparwasser et al., 1997). Bacterial DNA and ODN may also be the cause of inflammatory processes which are a common complication in lung infections (Schwartz et al., 1997). Furthermore, it is suspected that early gene therapy trials,
where formulated or non-formulated plasmid DNA-containing non-methylated CpG motifs was administered to the lung of cystic fibrosis patients failed, because of strong inflammatory processes, which were shown to be caused by CpG motifs (Paillard, 1999; Yew et al., 1999). CpG sequences contained in plasmid DNA also appear to be responsible for deaths of animals following intravenous injections of plasmid DNA formulated with liposomes (Paillard, 1999). Finally, animals exposed to high concentrations of CpG ODN develop symptoms of arthritis, likely due to inflammatory processes caused by the ODN (Deng et al., 1999).

Collectively, these reports highlight drawbacks of ODN adjuvants, which may be circumvented if the amount of ODNs to be used for a vaccine can be lowered significantly.

Polycationic polymers, for example the polycationic amino acid polymers poly-L-arginine and poly-L-lysine, have been shown to allow very efficient charging of antigen presenting cells (APCs) with antigens in vitro and in vivo (Buschle et al., Gene Ther Mol Biol 1, (1998), 309-321; Buschle et al., Proc Natl Acad Sci USA 94, (1997), 3256-3261; Schmidt et al., Proc Natl Acad Sci USA 94, (1997), 3262-3267). This is thought to be the key event for triggering immune cascades, eventually leading to the induction of antigen specific immune effector cells that are able to destroy or neutralize targets. It has been shown previously that a number of polycationic compounds exert effects on immune cells (Buschle et al., Gene Ther Mol Biol 1, (1998), 309-321; Buschle et al., Proc Natl Acad Sci USA 94, (1997), 3256-3261).

Co-injection of a mixture of poly-L-arginine or poly-L-lysine together with an appropriate antigen as a vaccine protect animals from tumor growth in several animal models (Buschle et al., Gene Ther Mol Biol 1, (1998), 309-321; Schmidt et al., Proc Natl Acad Sci USA 94, (1997), 3262-3267). Thus, a vaccine consisting of polycationic compounds and antigen(s) is accepted in the art as being a very effective form of treatment.

Mc Cluskie and Davies (Critical Reviews in Immunology 19 (1999), 303-329) describe the use of unmethylated immunostimulatory CpG ODNs for the induction of mucosal immunity. Mc Cluskie et al. (Vaccine 18 (2000), 231-237) disclose the administration of purified hepatitis B surface antigen with CpG ODN with cholene toxin to mice. The amino acid sequence of cholene toxin is disclosed in Dallas et al. (Nature 288 (1980), 499-501).

It is the object of the present invention to provide a pharmaceutical composition which allows an effective delivery to a target cell especially to the cellular immune system, but also to other cell types in order to induce potent immune responses. It is a further object of the invention to provide means to decrease or even ablate undesired immune responses.

This objective is solved by a pharmaceutical composition comprising
- an antigen or an immunosuppressing agent
- an immunogenic ODN, containing CpG motifs, and
- a polycationic peptide containing more than 20% of basic amino acids.

Surprisingly, it has turned out that the combination of the immunogenic ODN which may also include chemotactic or differentation inducing factor as immunostimulating substance and the polycationic peptide with an antigen according to the present invention leads to a synergistic immunomodulating effect for a given antigen preparation. The combination of antigen, immunogenic ODN and polycationic peptide allowed the generation of superior vaccines as compared to vaccines consisting of immunogenic ODN and antigen or antigen and polycationic peptide. In fact, this was observed even with suboptimal amounts of the immunogenic ODNs. Although such ODNs as well as the polycationic peptides have been known in the art (as well as many other substances for which an adjuvant effect is reported) as being potent factors in antigen preparations, the combination of these substances shows an effect which is by far better then only the combination of the single isolated effects of the compounds. In contrast to other combinations of different classes of adjuvants, where - at least - only additive effects are observed (if it all), the selection of the polycationic peptides and the immunogenic ODNs in a combined application together with an antigen showed an unforeseeable increase of effectiveness in immune response to a selected antigen. In a synergistic manner, the immunogenic ODNs and the polycationic polymers allow a very efficient cellular immune response as well as an immunomodulating effect which enables a superior vaccination perspective.

The administration of ODNs with CpG motifs also resulted in the induction of adverse side reactions, especially in the systemic release of pro-inflammatory cytokines, like TNF-α and IL-6. It was very surprising that these side effects can be prevented by providing a polycationic polymer together with the ODN and the antigen. Especially polycationic polymers comprising peptide bonds show an almost complete prevention of TNF-α and IL-6.

The antigens to be used in the present compositions are not critical. A vaccine can contain a whole variety of different antigens. Examples of antigens are whole-killed organisms such as inactivated viruses or bacteria, fungi, protozoa or even cancer cells. Antigens may also consist of subfractions of these organisms/tissues, of proteins, or, in their most simple form, of peptides. Antigens can also be recognized by the immune system in form of glycosylated proteins or peptides and may also be or contain polysaccharides or lipids. Short peptides can be used since for example cytotoxic T cells (CTL) recognize antigens in form of short usually 8-11 amino acids long peptides in conjunction with major histocompatibility complex (MHC) (Rammensee et al., Immunogenetics 41, (1995), 178-228). B cells recognize longer peptides starting at around 15 amino acids (Harlow et al, Cold Spring Harbor: Cold Spring Harbor Laboratory, (1988)). By contrast to T cell epitopes, the three dimensional structure of B cell antigens may also be important for recognition by antibodies. In order to obtain sustained, antigen-specific immune responses, adjuvants may help to trigger immune cascades that involve all cells of the immune system necessary. Primarily, adjuvants are acting, but are not restricted in their mode of action, on so-called antigen presenting cells (APCs). These cells usually first encounter the antigen(s) followed by presentation of processed or unmodified antigen to immune effector cells. Intermediate cell types may also be involved. Only effector cells with the appropriate specificity are activated in a productive immune response. The adjuvant may also locally retain antigens and co-injected other factors. In addition the adjuvant may act as a chemoattractant for other immune cells or may act locally and/or systemically as a stimulating agent for the immune system.

Preferably proteins or peptides derived from a viral or a bacterial pathogen or from fungi or parasites are used as such antigens (including derivatized antigens or glycosylated or lipidated antigens or polysaccharides or lipids). Another preferred source of antigens are tumor antigens. Preferred pathogens are selected from human immunodeficiency virus (HIV), hepatitis A and B viruses, hepatitis C virus (HCV), rous sarcoma virus (RSV), Epstein Barr virus (EBV) Influenza virus, Rotavirus, Staphylococcus aureus, Chlamydia pneumoniae, Chlamydia trachomatis, Mycobacterium tuberculosis, Streptococcus pneumoniae, Bacillus anthracis, Vibrio cholerae, Plasmodium sp. (Pl. falciparum, Pl. vivax, etc.), Aspergillus sp. or Candida albicans. Antigens may also be molecules expressed by cancer cells (tumor antigens). The derivation process may include the purification of a specific protein from the pathogen/cancer cells, the inactivation of the pathogen as well as the proteolytic or chemical derivatization or stabilization of such a protein. In the same way also tumor antigens (cancer vaccines) or autoimmune antigens may be used in the pharmaceutical composition according to the present invention. With such compositions a tumor vaccination or a treatment for autoimmume diseases may be performed.

In the case of peptide antigens the use of peptide mimitopes/agonists/superagonists/antagonists or peptides changed in certain positions without affecting the immunologic properties or non-peptide mimitopes/agonists/superagonists/antagonists (reviewed in Sparbier and Walden, 1999) is included in the current invention. Peptide antigens may also contain elongations either at the carboxy or at the amino terminus of the peptide antigen facilitating interaction with the polycationic compound(s) or the immunostimulatory compound(s). For the treatment of autoimmune diseases peptide antagonists may be applied.

Antigens may also be derivatized to include molecules enhancing antigen presentation and targeting of antigens to antigen presenting cells.

In one embodiment of the invention the pharmaceutical composition serves to confer tolerance to proteins or protein fragments and peptides which are involved in autoimmune diseases. Antigens used in this embodiments serve to tolerize the immune system or downregulate immune responses against epitopes involved in autoimmune processes.

The immunogenic ODN according to the present invention can be of prokaryotic and eukaryotic origin. In the case of eukaryotic origin, DNA should be derived from, based on the phylogenetic tree, less developed species (e.g. insects, but also others). In a preferred embodiment of the invention the immunogenic ODN is a synthetically produced DNA-molecule or mixtures of such molecules. Derivates or modifications of ODNs such as thiophosphate substituted analogues (thiophosphate residues substitute for phosphate) as for example described in US patents US 5,723,335 and US 5,663,153, and other derivatives and modifications, which preferably stabilize the immunostimulatory composition(s) but do not change their immunological properties (e.g. Sparbier and Walden, 1999), are also included. A preferred sequence motif is a six base DNA motif containing an (unmethylated) CpG dinucleotide flanked by two 5' purines and two 3' pyrimidines (5'-Pur-Pur-C-G-Pyr-Pyr-3') (Pisetsky, 1999). The CpG motifs contained in the ODNs according to the present invention are more common in microbial than higher vertebrate DNA and display differences in the pattern of methylation. (Bird, A.P., Nature 1986, 321:209; Pisetsky, D.S., Immunol Res 1999, 19 (1): 35-46). (Lipford et al., 1998). Surprisingly, sequences stimulating mouse APCs are not very efficient for human cells (Hartmann et al., 1999; Krieg, 1999). Preferred ODNs according to the present invention are disclosed e.g. in EP 0 468 520 A2, WO 96/02555, WO 98/16247, WO 98/18810, WO 98/37919, WO 98/40100, WO 98/52581, WO 98/52962, WO 99/51259 and WO 99/56755. Apart from stimulating the immune system certain ODNs are neutralizing some immune responses (Krieg, 1999; Lipford et al., 1998). These sequences are also included in the current invention, for example for applications for the treatment of autoimmune diseases.

ODNs/DNAs may be produced chemically or recombinantly or may be derived from natural sources. Preferred natural sources are insects. Of course, also mixtures of different ODNs may be used according to the present invention.

The polycationic peptide(s) to be used according to the present invention may be any polycationic peptide which shows the characteristic effect according to the WO 97/30721. Preferred polycationic peptides are selected from basic polypeptides, organic polycations, basic polyaminoacids or mixtures thereof. These polyaminoacids should have a chain length of at least 4 amino acid residues (see: Tuftsin as described in Goldman et al (1983)). Especially preferred are substances containing peptidic bonds, like polylysine, polyarginine and polypeptides containing more than 50% of basic amino acids in a range of more than 8, especially more than 20, amino acid residues or mixtures thereof. Other preferred polycations and their pharmaceutical compositons are described in WO 97/30721 (e.g. polyethyleneimine) and WO 99/38528. Preferably these polypeptides contain between 20 and 500 amino acid residues, especially between 30 and 200 residues.

These polycationic peptides may be produced chemically or recombinantly or may be derived from natural sources.

Cationic (poly)peptides may also be polycationic anti-bacterial microbial peptides with properties as reviewed in (Ganz and Lehrer, 1999; Hancock, 1999). These (poly)peptides may be of prokaryotic or animal or plant origin or may be produced chemically or recombinantly (Andreu and Rivas, 1998; Ganz and Lehrer, 1999; Simmaco et al., 1998). Peptides may also belong to the class of defensins (Ganz, 1999; Ganz and Lehrer, 1999). Sequences of such peptides can be, for example, be found in Tossi et al., Biopolymers (Peptide Science) 55 (2000), 4-30.

Such host defense peptides or defensives are also a preferred form of the polycationic peptide according to the present invention. Generally, a compound allowing as an end product activation (or down-regulation) of the adaptive immune system, preferably mediated by APCs (including dendritic cells) is used as polycationic peptide.

Especially preferred for use as polycationic substance in the present invention are cathelicidin derived antimicrobial peptides or derivatives thereof (A 1416/2000), especially antimicrobial peptides derived from mammal cathelicidin, preferably from human, bovine or mouse.

Polycationic peptides derived from natural sources include HIV-REV or HIV-TAT (derived cationic peptides, antennapedia peptides, chitosan or other derivatives of chitin) or other peptides derived from these peptides or proteins by biochemical or recombinant production. Other preferred polycationic compounds are cathelin or related or derived substances from cathelin. For example, mouse cathelin is a peptide which has the amino acid sequence NH₂-RLAGLLRKGGEKIGEKLKKIGOKIKNFFOKLVPOPE-COOH. Related or derived cathelin substances contain the whole or parts of the cathelin sequence with at least 15-20 amino acid residues. Derivations may include the substitution or modification of the natural amino acids by amino acids which are not among the 20 standard amino acids. Moreover, further cationic residues may be introduced into such cathelin molecules. These cathelin molecules are preferred to be combined with the antigen and the immunogenic ODN according to the present invention. However, these cathelin molecules surprisingly have turned out to be also effective as an adjuvant for a antigen without the addition of further adjuvants. It is therefore possible to use such cathelin molecules as efficient adjuvants in vaccine formulations with or without further immunactivating substances.

Another preferred polycationic substance to be used according to the present invention is a synthetic peptide containing at least 2 KT.,K-motifs separated by a linker of 3 to 7 hydrophobic amino acids (A 1789/2000).

It was very surprising that with the pharmaceutical composition according to the present invention the immunostimulating effect was significantly higher than it could be expected from the addition of the effects of each single component or even the addition of the effects of the polycation with the antigen and the immunogenic ODN with the antigen. Moreover, it turned out that the effect of the immunogenic ODN alone is not very high when an antigen is directly applied with this substance. This is true in particular if the compounds are not repeatedly administered. Very importantly combination of the compounds allows to use less of the immunogenic ODN which may help to avoid side effects (see above). Surprisingly, also side effects being present when ODNs are administered are prevented or reduced by the combined administration of antigen, ODN and polycationic peptide.

According to another aspect the present invention also relates to vaccines which comprise a composition according to the present invention.

Moreover, the present invention is also drawn to the use of the composition according to the present invention for manufacturing a vaccine.

The relative amounts of the ingredients of the present composition are highly depending on the necessities of the individual composition, e.g. the polycationic peptide to be used. In the case of poly-L-arginine and poly-L-lysine, preferred amounts of antigen/immunogenic ODN/immunosuppressive compound polycation lie in the range of 1-10000 µg antigen per vaccination, 1pM - 1mM immunogenic ODN per dose, and 0,1 to 1000 µg polycation per vaccination.

It is especially preferred to provide CpG ODNs and polycationic peptides (especially polycationic polypeptides, such as poly-arginine or poly-lysine) in a charge ratio of between 100:1 and 0,1:1, preferably between 50:1 and 0,5:1, especially between 10:1 and 0,5:1.

The present compositions may be applied to a patient, e.g. a vaccination candidate, in efficient amounts e.g. by weekly, biweekly or monthly intervals. Patients to be treated with the present compositions may also be vaccinated repeatedly or only once. A preferred use of the present invention is the active immunisation, especially of humans or animals without protection against the specific antigen.

The route of application for the present composition is not critical, e.g. subcutaneous, intramuscular, intradermal or transdermal injection is suitable as well as oral uptake. The adaption of the present composition to such an application route is easily conducted by the man skilled in the art.

It is also possible to apply the present composition separatedly e.g. by injecting the immunogenic ODN separatedly from the antigen/polycation composition. The present invention is therefore also directed to a kit comprising a composition containing the antigen and the polycationic peptide as one component and a composition containing the immunogenic ODN substance as a second component.

The components may be applied at the same site or time, however, an application at different sites or at a different time or for a different time period is also possible. It is also possible to vary the systemic or local applications of the composition or the components, respectively.

Another aspect of the present invention relates to a kit comprising a component containing an immunogenic ODN, a component containing a polycationic peptide containing more than 20% of basic amino acids and a component containing an antigen. Preferably the antigen is provided already mixed with the polycationic peptide.

The invention will be described in more detail by way of the following examples and the drawing figures, yet it is not restricted to these particular embodiments.
Fig. 1A and 1B: the IFN-γ-ELISPOT of the immune response against the OVA-peptide SIINFEKL;
Fig. 2: the IFN-γ-ELISPOT of the immune response against the mouse mastocytoma P815-derived peptide P1A (Fig. 2A), the CSP-peptide SYVPSAEQI (Fig. 2B), the LLO peptide GYKDGNEYI (Fig. 2C) and the OVA peptide ISQAVHAAHAEINE (Fig. 2D);
Fig. 3: the IFN-γ-ELISPOT of the immune response against the MC1R peptide WGPFFLHL;
Fig. 4: the IFN-γ-ELISPOT of the immune response at different injection sites against the OVA-peptide SIINFEKL; and

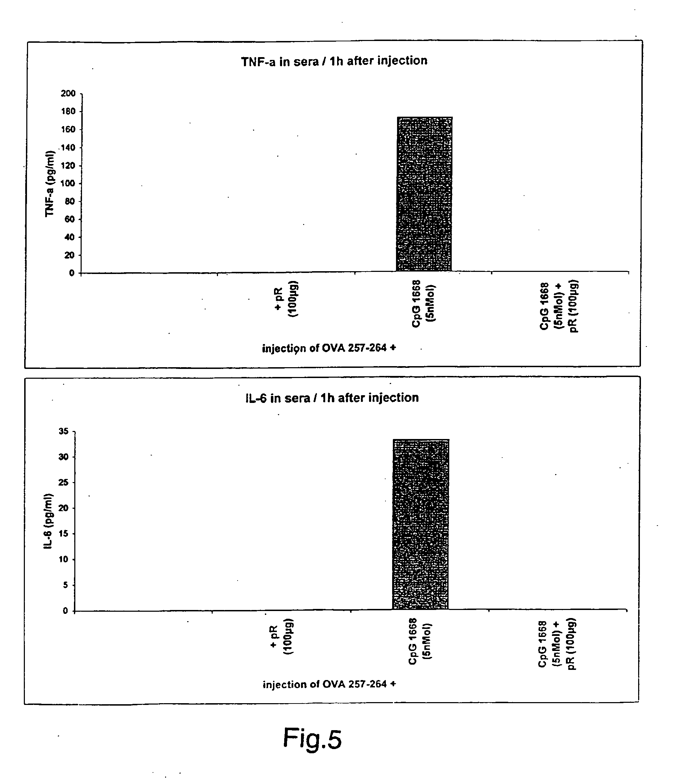

Fig. 5: the combined application of CpG-ODNs and poly-L-arginine (pR 60) prevents the induction of systemic TNF-α and IL-6 production.

### EXAMPLES

In all experiments thiophosphate-substituted ODNs (with thiophosphate residues substituting for phosphate, hereafter called "thiophosphate substituted oligodeoxynucleotides") were used since such ODNs display higher nuclease resistance (Ballas et al., 1996; Krieg et al., 1995; Parronchi et al., 1999).

Lymph nodes were passed through a 70 µm cell strainer and washed twice with DMEM medium (GIBCO BRL) containing 5% fetal calf serum (FCS, SIGMA chemicals). Cells were adjusted to 10⁷ cells/ml in DMEM/5%FCS. IFN-γ-ELISPOT assay were carried out in duplicates as described (Miyahira et al., 1995). This method is a widely used procedure allowing the quantification of antigen-specific T cells. Lymphocytes were stimulated ex vivo in duplicates with medium (background), pR 60, CpG-ODN and Concanavalin A (Con A). Spots representing single IFN-γ producing T cells were counted and the number of background spots was substracted from all samples. There were many spots detected after the stimulation with Con A (data not shown) indicating a good condition of the used lymphocytes.

**Example 1**: The combined application of CpG-ODN and poly-L-arginine (pR 60) strongly enhances the induction of Ovalbumin-peptide specific T cells in a concentration (pR 60)-dependent manner.

| | |
|---|---|
| Mice | C57Bl/6 (Harlan/Olac) |
| Peptide | OVA₂₅₇₋₂₆₄-Peptide (SIINFEKL), a MHC class I (H-2Kb)- |
| | restricted epitope of chicken ovalbumin (Rotzschke et al., 1991), was synthesized using standard solid phase F-moc synthesis, HPLC purified and analysed by mass spectroscopy for purity. Dose: 300 µg/mouse |
| Poly-L-Arginine 60 (pR60) | Poly-L-Arginine with an average degree of polymerization of 60 arginine residues; SIGMA chemicals Dose: 1000, 100 or 10 µg/mouse |
| CpG-ODN 1668 | thiophosphate substituted ODNs containing a CpG motif: TCC ATG ACG TTC CTG ATG CT, synthesized by NAPS GmbH, Göttingen. Dose: 5 nmol/mouse |
| Non-CpG-ODN 1911 | phosphothioates modified oligodinucleotides containing no CpG motif: TCC AGG ACT TTC CTC AGG TT, synthesized by NAPS GmbH, Göttingen. Dose: 5 nmol/mouse |

### Experimental groups (5 mice per group)

1. OVA₂₅₇₋₂₆₄-Peptide + CpG-ODN
2. OVA₂₅₇₋₂₆₄-Peptide + CpG-ODN + pR 60 1000 µg
3. OVA₂₅₇₋₂₆₄-Peptide + CpG-ODN + pR 60 100 µg
4. OVA₂₅₇₋₂₆₄-Peptide + CpG-ODN + pR 60 10 µg
5. OVA₂₅₇₋₂₆₄-Peptide + Non-CpG-ODN
6. OVA₂₅₇₋₂₆₄-Peptide + Non-CpG-ODN + pR 60 1000 µg
7. OVA₂₅₇₋₂₆₄-Peptide + Non-CpG-ODN + pR 60 100 µg
8. OVA₂₅₇₋₂₆₄-Peptide + Non-CpG-ODN + pR 60 10 µg
9. OVA₂₅₇₋₂₆₄-Peptide + pR 60 1000 µg
10. OVA₂₅₇₋₂₆₄-Peptide + pR 60 100 µg
11. OVA₂₅₇₋₂₆₄-Peptide + pR 60 10 µg

On day 0 mice were injected into each hind footpad with a total volume of 100 µl (50 µl per footpad) containing the above mentioned compounds. Animals were sacrificed 4 days after injection and popliteal lymph nodes were harvested. Lymph nodes were passed through a 70 µm cell strainer and washed twice with DMEM medium (GIBCO BRL) containing 5% fetal calf serum (FCS, SIGMA chemicals). Cells were adjusted to 10⁷ cells/ml in DMEM/5%FCS.

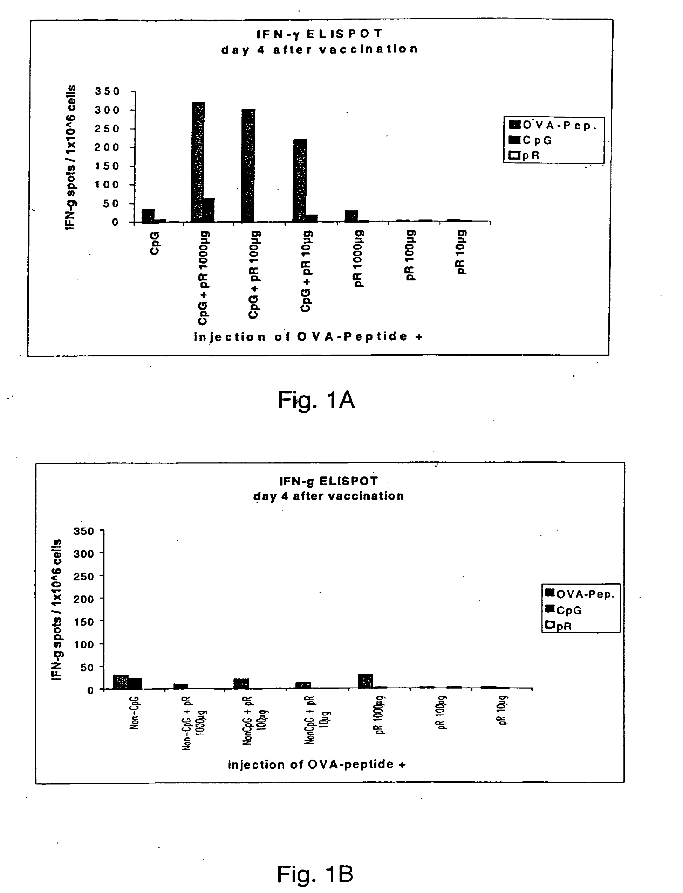

IFN-γ-ELISPOT assay were carried out in duplicates as described (Miyahira et al., 1995). This method is a widely used procedure allowing the quantification of antigen-specific T cells. Lymphocytes were stimulated ex vivo in duplicates with medium (background), pR 60, CpG-ODN and Concanavalin A (Con A). Spots representing single IFN-γ producing T cells were counted and the number of background spots was substracted from all samples. There were many spots detected after the stimulation with Con A (data not shown) indicating a good condition of the used lymphocytes. For each experimental group of mice the number of spots/1x10⁶ cells are illustrated in Figure 1A and 1B.

While the injection of OVA₂₅₇₋₂₆₄-peptide with poly-L-arginine or CpG-ODN alone leads to low numbers of peptide-specific IFN-γ-producing cells, the injection of OVA₂₅₇₋₂₆₄-peptide with the combination of poly-L-arginine and CpG-ODN strongly enhances the peptide-specific response. Using the non-immunogenic Non-CpG-ODN instead of CpG-ODN, the co-application of poly-L-arginine has no increasing effect on the peptide-specific immune response which is rather low.

**Example 2** : The combined application of CpG-ODN and poly-L-arginine strongly enhances the induction of T cells specific for a mastocytoma-derived peptide, a circum-sporozoite-derived peptide, a listeriolysin-derived peptide and a MHC class II restricted ovalbumin-derived peptide.

| | |
|---|---|
| Mice | DBA/2 (Harlan/Olac) |
| Peptides | a. Mouse mastocytoma P815-derived peptide P1A (LPYLGWLVF), restricted to MHC class I (H2-Ld) (Lethe et al., 1992). |
| | b. CSP-peptide (SYVPSAEQI) derived from the circumsporozoite protein of plasmodium yoelii (Rodrigues et al., 1992), restricted to MHC class I (H2-Kd). |
| | c. LLO-peptide (GYKDGNEYI) derived from listeriolysin O 91-99 of Listeria monocytogenes (Pamer et al., 1991), restricted to MHC class I (H2-Kd). |
| | d. OVA₃₂₃₋₃₃₆-Peptide (ISQAVHAAHAEINE) derived from |
| | chicken ovalbumin, restricted to MHC class II (I-Ad) (Shimonkevitz et al., 1984). All peptides were synthesized by standard solid phase F-moc synthesis, HPLC purified and analysed by mass spectroscopy for purity. Dose: 300 µg/mouse |
| Poly-L-Arginine 60 (pR60) | Poly-L-Arginine with an average degree of polymerization of 60 arginine residues; SIGMA chemicals Dose: 100 µg/mouse |
| CpG-ODN 1668 | thiophosphate substituted ODNs containing a CpG motif: TCC ATG ACG TTC CTG ATG CT, synthesized by NAPS GmbH, Göttingen. Dose: 5 nmol/mouse |
| Non-CpG-ODN 1911 | thiophosphate substituted ODNs containing no CpG motif: TCC AGG ACT TTC CTC AGG TT, synthesized by NAPS Göttingen GmbH. Dose: 5 nmol/mouse |

### Experimental groups (5 mice per group)

1. P1A-Peptide + CpG-ODN + pR 60 100 µg
2. P1A-Peptide + Non-CpG-ODN + pR 60 100 µg
3. P1A-Peptide + CpG-ODN
4. P1A-Peptide + pR 60 100 µg

5. CSP-Peptide + CpG-ODN + pR 60 100 µg
6. CSP-Peptide + Non-CpG-ODN + pR 60 100 µg
7. CSP-Peptide + CpG-ODN
8. CSP-Peptide + pR 60 100 µg
9. LLO-Peptide + CpG-ODN + pR 60 100 µg

10. LLO-Peptide + Non-CpG-ODN + pR 60 100 µg
11. LLO-Peptide + CpG-ODN
12. LLO-Peptide + pR 60 100 µg

13. OVA-Peptide + CpG-ODN + pR 60 100 µg
14. OVA-Peptide + Non-CpG-ODN + pR 60 100 µg
15. OVA-Peptide + CpG-ODN
16. OVA-Peptide + pR 60 100 µg

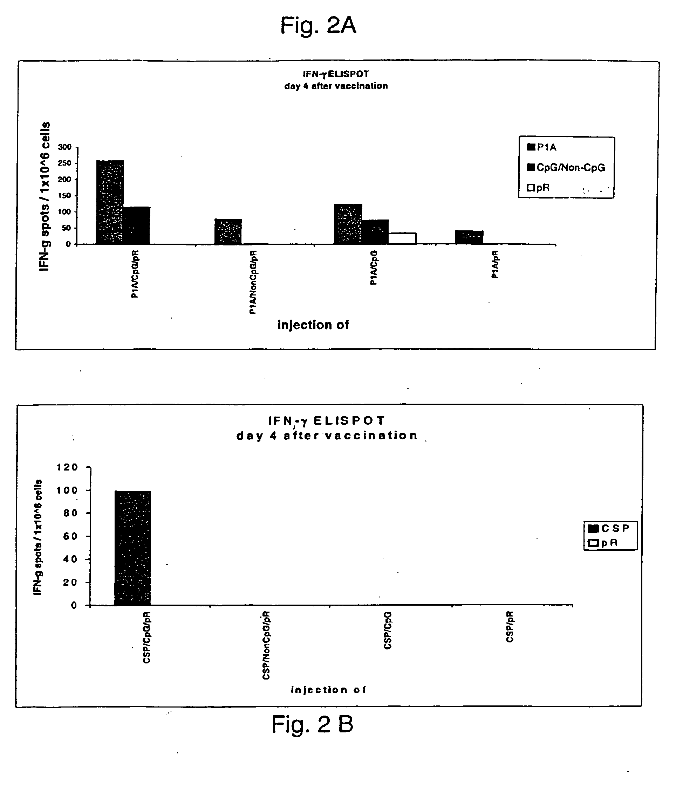

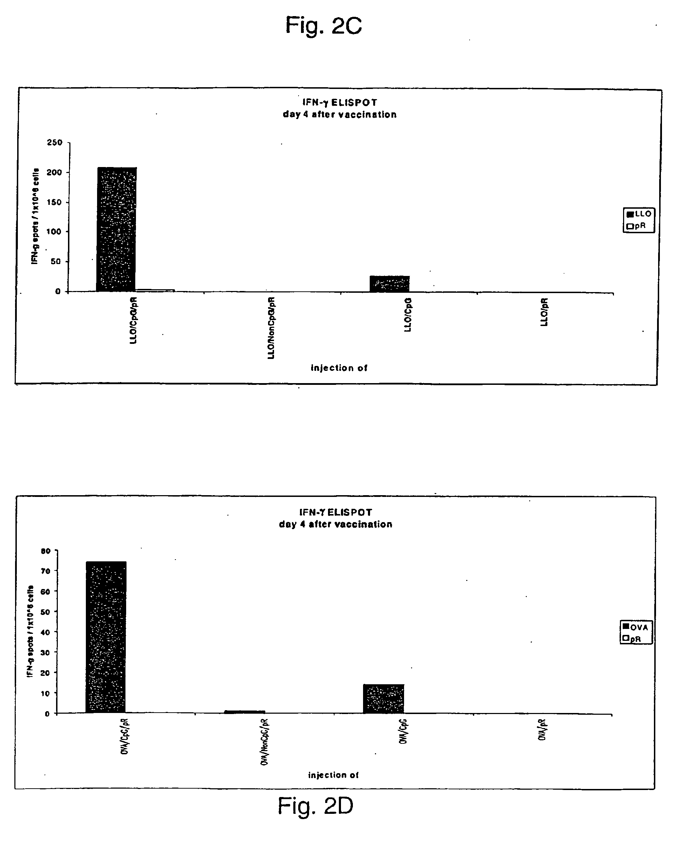

On day 0 mice were injected into each hind footpad with a total volume of 100 µl, 50 µl per footpad, containing the above mentioned compounds. Animals were sacrificed 4 days after injection and popliteal lymph nodes were harvested. Lymph nodes and (Shimonkevitz et al., 1984) were prepared as described in example 1 and IFN-γ-ELISPOTs were prepared. For each experimental group of mice the number of spots/1x10⁶ cells are illustrated in Figure 2A-D.

While the injection of the peptides with poly-L-arginine or CpG-ODN alone leads to no or only low numbers of peptide-specific IFN-γ-producing cells, the injection of peptides with the combination of poly-L-arginine and CpG-ODN induces (e.g. CSP) or strongly enhances the peptide-specific response. Using the non-immunogenic Non-CpG-ODN instead of CpG-ODN, the co-application of poly-L-arginine has no increasing effect on the peptide-specific immune response which is rather low.

**Example 3** : The combined application of CpG-ODN and poly-L-arginine (pR 60) synergistically enhances the immune response against MC1R (melanocyte stimulating hormone receptor).

| | |
|---|---|
| Mice | C57B1/6 (Harlan/Olac) |
| Peptide | MC1R-peptide (WGPFFLHL, F. Mattner, not published), a MHC class I (H-2Kb)-restricted epitope of melanocyte stimulating hormone receptor (MC1R) synthesized by standard solid phase F-moc synthesis, HPLC purified and analysed by mass spectroscopy for purity. Dose: 300 µg/mouse |
| Poly-L-Arginine 60 (pR60) | Poly-L-Arginine with an average degree of polymerization of 60 arginine residues; SIGMA chemicals Dose: 1000, 100 or 10 µg/mouse |
| CpG-ODN 1668 | thiophosphate substituted containing a CpG motif: TCC ATG ACG TTC CTG ATG CT, synthesized by NAPS Göttingen GmbH. |
| Dose: | 5 nmol/mouse |

### Experimental groups (3 mice per group)

1. MC1R + CpG-ODN + pR
2. MC1R + CpG-ODN
3. MC1R + pR

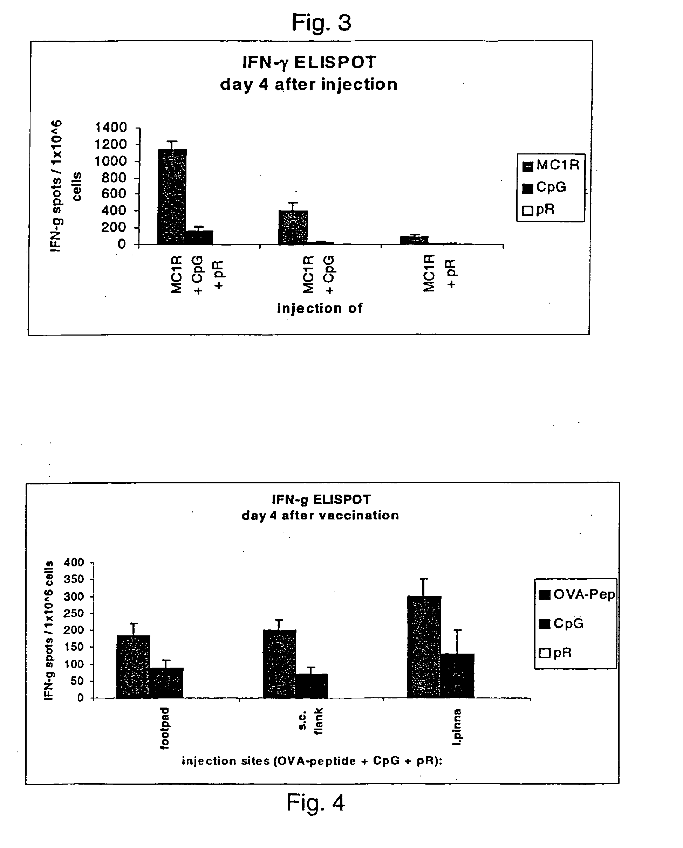

At day 0 mice were injected into each hind footpad with a total volume of 100 µl (50 µl per footpad) containing the above mentioned compounds. Animals were sacrificed 4 days after injection and popliteal lymph nodes were harvested. Lymph nodes and IFN-γ-ELISPOTs were prepared as described in example 1. For each experimental group of mice the number of spots/1x10⁶ cells are illustrated in Figure 3, standard deviation of ex vivo-stimulated triplicates were given.

While the injection of MC1R-peptide with poly-L-arginine or CpG-ODN alone leads to low numbers of peptide-specific IFN-γ-producing cells, the injection of MC1R-peptide with the combination of poly-L-arginine and CpG-ODN strongly enhances the peptide-specific response. ,

**Example 4** : The combined application of CpG-ODN and poly-L-arginine (pR 60) induces at different injection sites a strong antigen specific immune response against Ovalbumin-peptide.

| | |
|---|---|
| Mice | C57Bl/6 (Harlan/Olac) |
| Peptide | OVA₂₅₇₋₂₆₄-Peptide (SIINFEKL), a MHC class I (H-2Kb)-restricted epitope of chicken Ovalbumin (Rotzschke et al., 1991), synthesized by standard solid phase F-moc synthesis, HPLC purified and analysed by mass spectroscopy for purity. Dose: 300 µg/mouse |
| Poly-L-Arginine 60 (pR60) | Poly-L-Arginine with an average degree of polymerization of 60 arginine residues; SIGMA chemicals Dose: 100 µg/mouse |
| CpG-ODN 1668 | thiophosphate substituted ODNs containing a CpG motif: TCC ATG ACG TTC CTG ATG CT, synthesized |
| | by NAPS Göttingen GmbH. |
| | Dose: 5 nmol/mouse |

### Experimental groups (5 mice per group) injection sites:

1. footpad
2. s.c. /flank
3. intra pinna

At day 0 mice were injected (Ovalbumin-peptide + CpG-ODN + pR 60) into the different injection sites with a total volume of 100 µl (50 µl per footpad) containing the above mentioned compounds. Animals were sacrificed 4 days after injection and the draining lymph nodes were harvested. Lymph nodes and IFN-γ- ELISPOTs were prepared in triplicates (standard deviation is given) as described in example 1. For each experimental group of mice the number of spots/1x10⁶ cells are illustrated in Figure 4.

The co-application of OVA₂₅₇₋₂₆₄-peptide with poly-L-arginine and CpG-ODN induces at different injection sites a strong antigen specific immune response. The intra pinna injection is superior to the other subcutaneous injections (footpad, flank).

**Table 2**

| **Peptide antigens used for vaccination experiments** | | | | |
|---|---|---|---|---|
| ***peptide*** | ***sequence*** | ***source*** | ***restricted to*** | ***publication*** |
| OVA₂₅₇₋₂₆₄ | SIINFEKL | Chicken Ovalbumin | MHC class I, H2Kb | (Rotzschke et al., 1991) |
| P1A | LPYLGWLVF | Mouse mastocytoma P815 | MHC class I, H-2Kd | (Lethe et al., 1992) |
| CSP | SYVPSAEQI | Circumsporozoite protein Plasmodium yoelii | MHC class I, H-2Kd | (Rodrigues et al., 1992) |
| LLO | GYKDGNEYI | Listeriolysin Listeria monocytogenes | MHC class I, H2kd | (Pamer et al., 1991) |
| OVA₃₂₃₋₃₃₆ | ISQAVHAA-HAEINE | Chicken Ovalbumin | MHC class II, I-Ad | (Shimonkevitz et al., 1984) |
| MC1R | WGPFFLHL | melanocyte stimulating hormone receptor | MHC class I, H-2Kb | F. Mattner, not published |

**Example 5** : The combined application of CpG-ODNs and poly-L-arginine (pR 60) prevents the induction of systemic TNF-α and IL-6-production.

| | |
|---|---|
| Mice | C57B1/6 (Harlan/Olac) |
| Peptide | OVA₂₅₇₋₂₆₄-peptide (SIINFEKL, a MHC class I (H-2Kb)-restricted epitope of chicken ovalbumin (Rotzschke et al., 1991), was synthesized using standard solid phase F-moc synthesis, HPLC-purified and analysed by mass spectroscopy for purity Dose: 300 µg/mouse |
| Poly-L-Arginine 60 (pR60) | Poly-L-arginine with an average degree of polymerization of 60 arginine residues; SIGMA chemicals Dose: 100 µg/mouse |
| CpG-ODN 1668 | thiophosphate substituted ODNs containing a CpG motif: TCC ATG ACG TTC CTG ATG CT, synthesized by NAPS GmbH, Göttingen. Dose: 5 nmol/mouse |

### Experimental groups (4 mice per group)

1. OVA₂₅₇₋₂₆₄-peptide
2. pR60
3. CpG 1668 + OVA₂₅₇₋₂₆₄-peptide
4. CpG 1668 + pR60 + OVA₂₅₇₋₂₆₄-peptide

Mice were injected into each hind footpad with a total volume of 100 µl, 50 µl per footpad, containing the above mentioned compounds. One hour after injection blood was taken via the tailvein and serum was prepared. The amount of proinflammatory cytokines (TNF-α and IL-6) in the sera was determined using cytokine-specific ELISAs.

| **TNF-α/IL-6 in sera / 1 h after injection** | | |
|---|---|---|
| **injection 245+** | **TNF-α (pg/ml)** | **IL-6 (pg/ml)** |
| - | 0 | 0 |
| +pR (100 µg) | 0 | 0 |
| CpG 1668 (5nMol) | 171,5 | 33,1 |
| CpG 1668 (5nMol)+pR (100µg) | 0 | 0 |

### References:

Andreu, D., and Rivas, L. (1998). Animal antimicrobial peptides: an overview. Biopolymers 47, 415-433.
Ballas, Z. K., Rasmussen, W. L., and Krieg, A. M. (1996). Induction of NK activity in murine and human cells by CpG motifs in oligodeoxynucleotides and bacterial DNA. J Immunol 157, 1840-1845.
Bloom, B. R., and Widdus, R. (1998). Vaccine visions and their global impact. Nat Med 4, 480-484.
Chace, J. H., Hooker, N. A., Mildenstein, K. L., Krieg, A. M., and Cowdery, J. S. (1997). Bacterial DNA-induced NK cell IFN-gamma production is dependent on macrophage secretion of IL-12. Clin Immunol Immunopathol 84, 185-193.
Davis, H. L., Weeranta, R., Waldschmidt, T. J., Tygrett, L., Schorr, J., and Krieg, A. M. (1998). CpG DNA is a potent enhancer of specific immunity in mice immunized with recombinant hepatitis B surface antigen. J Immunol 160, 870-876.
Deng, G. M., Nilsson, I. M., Verdrengh, M., Collins, L. V., and Tarkowski, A. (1999). Intra-articularly localized bacterial DNA containing CpG motifs induces arthritis. Nat Med 5, 702-705.
Ganz, T. (1999). Defensins and host defense [comment]. Science 286, 420-421.
Ganz, T., and Lehrer, R. I. (1999). Antibiotic peptides from higher eukaryotes: biology and applications. Mol Med Today 5, 292-297.
Hancock, R. E. (1999). Host defence (cationic) peptides: what is their future clinical potential? Drugs 57, 469-473.
Harlow, E., and Lane, D. (1988). Antibodies: a laboratory manual (Cold Spring Harbor: Cold Spring Harbor Laboratory).
Hartmann, G., Weiner, G. J., and Krieg, A. M. (1999). CpG DNA: A potent signal for growth, activation, and maturation of human dendritic cells. Proc Natl Acad Sci U S A 96, 9305-9310.
Hoffmann, J. A., Kafatos, F. C., Janeway, C. A., and Ezekowitz, R. A. (1999). Phylogenetic perspectives in innate immunity. Science 284, 1313-1318.
Klinman, D. M., Yi, A. K., Beaucage, S. L., Conover, J., and Krieg, A. M. (1996). CpG motifs present in bacteria DNA rapidly induce lymphocytes to secrete interleukin 6, interleukin 12, and interferon gamma. Proc Natl Acad Sci U S A 93, 2879-2883.
Krieg, A. M. (1999). CpG DNA: a novel immunomodulator [letter]. Trends Microbiol 7, 64-5.
Krieg, A. M. (1996). An innate immune defense mechanism based on the recognition of CpG motifs in microbial DNA. J Lab Clin Med 128, 128-133.
Krieg, A. M., Yi, A. K., Matson, S., Waldschmidt, T. J., Bishop, G. A., Teasdale, R., Koretzky, G. A., and Klinman, D. M. (1995). CpG motifs in bacterial DNA trigger direct B-cell activation. Nature 374, 546-549.
Krieg, A. M., Yi, A. K., Schorr, J., and Davis, H. L. (1998). The role of CpG dinucleotides in DNA vaccines. Trends Microbiol 6, 23-27.
Lethe, B., van den Eynde, B., van Pel, A., Corradin, G., and Boon, T. (1992). Mouse tumor rejection antigens P815A and P815B: two epitopes carried by a single peptide. Eur J Immunol 22, 2283-2288.
Liljeqvist, S., and Stahl, S. (1999). Production of recombinant subunit vaccines: protein immunogens, live delivery systems and nucleic acid vaccines. J Biotechnol 73, 1-33.
Lipford, G. B., Heeg, K., and Wagner, H. (1998). Bacterial DNA as immune cell activator. Trends Microbiol 6, 496-500.
Mosmann, T. R., Cherwinski, H., Bond, M. W., Giedlin, M. A., and Coffman, R. L. (1986). Two types of murine helper T cell clone. I. Definition according to profiles of lymphokine activities and secreted proteins. J Immunol 136, 2348-2357.
Nossal, G. (1998). Living up to the legacy. Nat Med 4, 475-476.
Oxenius, A., Martinic, M. M., Hengartner, H., and Klenerman, P. (1999). CpG-containing oligonucleotides are efficient adjuvants for induction of protective antiviral immune responses with T-cell peptide vaccines. J Virol 73, 4120-4126.
Paillard, F. (1999). CpG: the double-edged sword [comment]. Hum Gene Ther 10, 2089-2090.
Pamer, E. G., Harty, J. T., and Bevan, M. J. (1991). Precise prediction of a dominant class I MHC-restricted epitope of Listeria monocytogenes. Nature 353, 852-855.
Parronchi, P., Brugnolo, F., Annunziato, F., Manuelli, C., Sampognaro, S., Mavilia, C., Romagnani, S., and Maggi, E. (1999). Phosphorothioate oligodeoxynucleotides promote the in vitro development of human allergen-specific CD4+ T cells into Th1 effectors. J Immunol 163, 5946-5953.
Pisetsky, D. S. (1997). Immunostimulatory DNA: a clear and present danger? Nat Med 3, 829-831.
Pisetsky, D. S. (1999). The influence of base sequence on the immunostimulatory properties of DNA. Immunol Res 19, 35-46.
Rodrigues, M., Nussenzweig, R. S., Romero, P., and Zavala, F. (1992). The in vivo cytotoxic activity of CD8+ T cell clones correlates with their levels of expression of adhesion molecules. J Exp Med 175, 895-905.
Roitt, I., Brostoff, J., and Male, D. (1998). Immunology (London: Mosby International Ltd).
Rotzschke, O., Falk, K., Stevanovic, S., Jung, G., Walden, P., and Rammensee, H. G. (1991). Exact prediction of a natural T cell epitope. Eur J Immunol 21, 2891-2894.
Schwartz, D. A., Quinn, T. J., Thorne, P. S., Sayeed, S., Yi, A. K., and Krieg, A. M. (1997). CpG motifs in bacterial DNA cause inflammation in the lower respiratory tract. J Clin Invest 100, 68-73.
Shimonkevitz, R., Colon, S., Kappler, J. W., Marrack, P., and Grey, H. M. (1984). Antigen recognition by H2-resctricted T cells II. A tryptic ovalbumin peptide that substitutes for processed antigen. J Immunol 133, 2067-2074.
Simmaco, M., Mignogna, G., and Barra, D. (1998). Antimicrobial peptides from amphibian skin: what do they tell us? Biopolymers 47, 435-450.
Sparbier, K., and Walden, P. (1999). T cell receptor specificity and mimotopes. Curr Opin Immunol 11, 214-218.
Sparwasser, T., Koch, E. S., Vabulas, R. M., Heeg, K., Lipford, G. B., Ellwart, J. W., and Wagner, H. (1998). Bacterial DNA and immunostimulatory CpG oligonucleotides trigger maturation and activation of murine dendritic cells. Eur J Immunol 28, 2045-2054.
Sparwasser, T., Miethke, T., Lipford, G., Borschert, K., Hacker, H., Heeg, K., and Wagner, H. (1997). Bacterial DNA causes septic shock [letter]. Nature 386, 336-337.
Sparwasser, T., Miethke, T., Lipford, G., Erdmann, A., Hacker, H., Heeg, K., and Wagner, H. (1997). Macrophages sense pathogens via DNA motifs: induction of tumor necrosis factor-alpha-mediated shock. Eur J Immunol 27, 1671-1679.
Weiner, G. J., Liu, H. M., Wooldridge, J. E., Dahle, C. E., and Krieg, A. M. (1997). Immunostimulatory oligodeoxynucleotides containing the CpG motif are effective as immune adjuvants in tumor antigen immunization. Proc Natl Acad Sci U S A 94, 10833-10837.
Yew, N. S., Wang, K. X., Przybylska, M., Bagley, R. G., Stedman, M., Marshall, J., Scheule, R. K., and Cheng, S..H. (1999). Contribution of plasmid DNA to inflammation in the lung after administration of cationic lipid:pDNA complexes. Hum Gene Ther 10, 223-234.

## Claims

1. Pharmaceutical composition comprising
- an antigen,
- an immunogenic oligodeoxynucleotide containing CpG motifs (CpG-ODN), and
- a polycationic peptide containing more than 20% of basic amino acids.

2. Composition according to claim 1, **characterized in that** the antigen is a protein derived from a viral, parasitic or bacterial pathogen.

3. Composition according to claim 1, **characterized in that** the antigen is a tumor antigen.

4. Composition according to claim 1, **characterized in that** the antigen is an autoimmune antigen.

5. Composition according to any one of claims 1 to 4, **characterized in that** the polycationic peptide is a basic polypeptide, an organic polycation, a basic polyaminoacid or mixtures thereof.

6. Composition according to any one of claims 1 to 5, **characterized in that** the polycationic peptide is polylysine, polyarginine, a polypeptide containing more than 50 % of basic amino acids in a range of more than 8, especially more than 20, amino acid residues or mixtures thereof.

7. Composition according to any one of claims 1 to 6, **characterized in that** the polycationic peptide is derived from the REV-protein or the TAT-protein of HIV, chitosan or other chitin derivatives.

8. Composition according to any one of claims 1 to 7, **characterized in that** the immunogenic CpG-ODN is selected from synthetically produced DNA molecules, thiophosphate substituted ODNs, immunogenic insect DNA, recombinant DNA molecules, DNA molecules containing a CpG dinucleotide flanked by two 5' purines and two 3' pyrimidines, or mixtures thereof.

9. Vaccine, comprising a composition according to any one of claims 1 to 8.

10. Use of a composition according to any one of claims 1 to 8 for manufacturing a vaccine.

11. Kit comprising
- a component containing an immunogenic CpG-ODN, and
- a component containing a polycationic peptide containing more than 20% of basic amino acids and
- a component containing an antigen.

## Patentansprüche

1. Pharmazeutische Zusammensetzung, welche umfasst:
- ein Antigen,
- ein immunogenes, CpG-Motive enthaltendes Oligodesoxynukleotid (CpG-ODN), und
- ein polykationisches Peptid, das mehr als 20% basische Aminosäuren enthält.

2. Zusammensetzung gemäß Anspruch 1, **dadurch gekennzeichnet, dass** das Antigen ein Protein ist, das von einem viralen, parasitären oder bakteriellen Pathogen abgeleitet ist.

3. Zusammensetzung gemäß Anspruch 1, **dadurch gekennzeichnet, dass** das Antigen ein Tumorantigen ist.

4. Zusammensetzung gemäß Anspruch 1, **dadurch gekennzeichnet, dass** das Antigen ein Autoimmunantigen ist.

5. Zusammensetzung gemäß einem der Ansprüche 1 bis 4, **dadurch gekennzeichnet, dass** das polykationische Peptid ein basisches Polypeptid, ein organisches Polykation, eine basische Polyaminosäure oder Mischungen davon sind.

6. Zusammensetzung gemäß einem der Ansprüche 1 bis 5, **dadurch gekennzeichnet, dass** das polykationische Peptid Polylysin, Polyarginin, ein Polypeptid, das mehr als 50% basische Aminosäuren in einem Bereich von mehr als 8, insbesondere mehr als 20 Aminosäureresten enthält, ist oder Mischungen davon.

7. Zusammensetzung gemäß einem der Ansprüche 1 bis 6, **dadurch gekennzeichnet, dass** das polykationische Peptid vom REV-Protein oder dem TAT-Protein von HIV, Chitosan oder anderen Chitinderivaten abgeleitet ist.

8. Zusammensetzung gemäß einem der Ansprüche 1 bis 7, **dadurch gekennzeichnet, dass** das immunogene CpG-ODN ausgewählt ist aus synthetisch hergestellten DNA-Molekülen, Thiophosphat-substituierten ODNs, immunogener Insekten-DNA, rekombinanten DNA-Molekülen, DNA-Molekülen, die ein CpG-Dinukleotid enthalten, das von zwei 5'-Purinen und zwei 3'-Pyrimidinen flankiert ist, oder Mischungen davon.

9. Vakzin, das eine Zusammensetzung gemäß einem der Ansprüche 1 bis 8 umfasst.

10. Verwendung einer Zusammensetzung gemäß einem der Ansprüche 1 bis 8 zur Herstellung eines Vakzins.

11. Set, welches umfasst:
- eine Komponente, die ein immunogenes CpG-ODN enthält, und
- eine Komponente, die ein polykationisches Peptid enthält, das mehr als 20% basische Aminosäuren enthält, und
- eine Komponente, die ein Antigen enthält.

## Revendications

1. Composition pharmaceutique comprenant
- un antigène,
- un oligodésoxynucléotide immunogène contenant des motifs CpG (CpG-ODN), et
- un peptide polycationique contenant plus de 20 % d'acides aminés basiques.

2. Composition selon la revendication 1, **caractérisée en ce que** l'antigène est une protéine dérivée d'un agent pathogène viral, parasitaire ou bactérien.

3. Composition selon la revendication 1, **caractérisée en ce que** l'antigène est un antigène tumoral.

4. Composition selon la revendication 1, **caractérisée en ce que** l'antigène est un antigène auto-immun.

5. Composition selon l'une quelconque des revendications 1 à 4, **caractérisée en ce que** le peptide polycationique est un polypeptide basique, un polycation organique, un poly(acide aminé) basique ou un mélange de ceux-ci.

6. Composition selon l'une quelconque des revendications 1 à 5, **caractérisée en ce que** le peptide polycationique est la polylysine, la polyarginine, un polypeptide contenant plus de 50 % d'acides aminés basiques sur une gamme de plus de 8, en particulier plus de 20, résidus d'acides aminés, ou un mélange de ceux-ci.

7. Composition selon l'une quelconque des revendications 1 à 6, **caractérisée en ce que** le peptide polycationique est dérivé de la protéine REV ou de la protéine TAT du VIH, du chitosane ou d'autres dérivés de la chitine.

8. Composition selon l'une quelconque des revendications 1 à 7, **caractérisée en ce que** le CpG-ODN immunogène est choisi parmi les molécules d'ADN produites par synthèse, les ODN substitués par du thiophosphate, l'ADN d'insecte immunogène, les molécules d'ADN recombinant, les molécules d'ADN contenant un dinucléotide CpG flanqué par deux purines en 5' et deux pyrimidines en 3', et les mélanges de ceux-ci.

9. Vaccin comprenant une composition selon l'une quelconque des revendications 1 à 8.

10. Utilisation d'une composition selon l'une quelconque des revendications 1 à 8 pour la fabrication d'un vaccin.

11. Kit comprenant
- un composant contenant un CpG-ODN immunogène, et
- un composant contenant un peptide polycationique contenant plus de 20 % d'acides aminés basiques, et
- un composant contenant un antigène.
